(19) Europäisches Patentamt
European Patent Office
Office européen des brevets

(11) EP 0 967 215 B1

(12) **EUROPÄISCHE PATENTSCHRIFT**

(45) Veröffentlichungstag und Bekanntmachung des Hinweises auf die Patenterteilung:
**02.04.2003 Patentblatt 2003/14**

(51) Int Cl.7: **C07F 1/00**, C07F 3/00, C07B 33/00, C07C 45/29

(21) Anmeldenummer: **99112025.4**

(22) Anmeldetag: **22.06.1999**

(54) **Cu(II)- und Zn(II)-Phenoxylkomplexe und Radikalkomplexe hiervon, Verfahren zu deren Herstellung und deren Verwendung**

Cu(II) and Zn(II)-phenoxy complexes, and radical complexes derived therefrom, their preparation and use

Phénoxy complexes de Cu(II) et Zn(II) et complexes radicales derivés de celles-ci, leur préparation et leur utilisation

(84) Benannte Vertragsstaaten:
**BE DE FI FR GB IT NL**

(30) Priorität: **26.06.1998 DE 19828492**
**02.06.1999 DE 19925142**

(43) Veröffentlichungstag der Anmeldung:
**29.12.1999 Patentblatt 1999/52**

(73) Patentinhaber: **Degussa AG**
**40474 Düsseldorf (DE)**

(72) Erfinder:
- **Hess, Martina**
  **45479 Mülheim/Ruhr (DE)**
- **Chaudhuri, Phalguni, Dr.**
  **37079 Göttingen (DE)**
- **Wieghardt, Karl, Prof.Dr.**
  **44879 Bochum (DE)**

(56) Entgegenhaltungen:
- **MAIYA, B.G. ET AL.: "charge distribution in schiff-base biquinojne complexes. electrochemical, spectroelectrochemical and electron spin resonance studies of complexes of co, ni, zn, cu, and cd with n-(2'-hydroxy-3',5'-di-t-butylphenyl)-4,6-di-t-butyl-o-benzoquinone imine ligand system in ch2cl2" JOURNAL OF THE CHEMICAL SOCIETY, DALTIN TRANSACTIONS, 1990, Seiten 3571-3576, XP002163901**
- **SIMPSON, C.L. ET AL.: "charge distribution in transition metal complexes of a schiff base biquinone ligand. structural and electrochemical properties of the mii(cat-n-bq)2, miii(cat-n-bq)(cat-n-sq), miv(cat-n-sq)2 tautomeric series" INORGANIC CHEMISTRY, 1989, Seiten 4379-4385, XP000982739**
- **SPEIERE, G. ET AL.: "studies on aerobic reactions of ammonia/3,5-di-tert-bytylcatechol schiff base condensation products with copper, copper(I), and copper(II). Strong copper(II) -radical ferromagnetic exchange and observations on a unique n-n coupling reaction" INORGANIC CHEMISTRY, 1996, Seiten 3519-3524, XP000982755**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 967 215 B1

## Beschreibung

Die Erfindung betrifft einkernige $Cu^{II}$- und $Zn^{II}$-Phenoxylkomplexe sowie ein- und zweikernige Radikalkomplexe hiervon, Verfahren zur Herstellung der Komplexe und die Verwendung der Phenoxylradikalkomplexe als Katalysator bei Oxidationsreaktionen mit Sauerstoff, insbesondere zur Oxidation von primären oder sekundären Alkoholen.

Es ist bekannt, Aldehyde durch Dehydrierung oder durch Oxidation von primären Alkoholen zu erzeugen; im ersten Fall entsteht als Nebenprodukt molekularer Wasserstoff, im zweiten Fall Wasser.

Unter Verwendung enzymatischer Redosysteme lassen sich verschiedene Substrate oxidieren, wobei sich als Koppelprodukt Wasserstoffperoxid bildet. Hierbei oxidiert die in der Regel flavin-abhängige Oxidase das Substrat, und die reduzierte Form des Enzyms wird durch molekularen Sauerstoff unter Bildung von Wasserstoffperoxid reoxidiert. Beispielsweise kann ein Oxidase-Aktivität aufweisendes Enzym, wie Glucose-Oxidase, gemäß DE-A 42 31 767 in Verbindung mit dem oxidierbaren Substrat als Bleichsystem in Bleich- und Waschmitteln Verwendung finden. Gemäß US-Patent 5,234,827 ist es möglich, unter Einsatz eines kurzkettigen Alkohols, einer extrazellulären Alkohol-Oxidase, die frei von Katalase-Aktivität ist, in wäßriger Phase Wasserstoffperoxid herzustellen. Nachteile der enzymatischen Systeme für Oxidationsreaktionen und/oder die Gewinnung von Wasserstoffperoxid sind die Einschränkungen bezüglich des zulässigen pH- und Temperaturbereichs, die hohe Substratspezifität, die einer vielseitigen Anwendung oft entgegensteht, und die Gefahr einer Desaktivierung und damit Reduzierung der Aktivität des Enzyms durch irreversible Oxidation mittels des gebildeten Wasserstoffperoxids.

Es ist bekannt, daß Galactose-Oxidase, welche die Oxidation primärer Alkohole zu Aldehyden und $H_2O_2$ katalysiert, in der aktiven Form ein $Cu^{II}$-Ion an ein in ortho-Stellung S-modifiziertes Tyrosylradikal gebunden enthält. Die Galactose-Oxidase besitzt damit einen radikalischen Cofaktor - siehe beispielsweise N. Ito et al. in J. Mol. Biol. (1994) 238, 794 und R.M. Wachter et al. in J. Amer. Chem. Soc. (1996), 118, 2782. Ausgehend von den $Cu^{II}$-Phenoxyl-Strukturmodellen für die aktive Form der Glucose-Oxidase wurden von Y. Wang, T.D.P. Stack et al. in Science (1998) 279, 537 mononukleare $Cu^{II}$-Verbindungen mit vierzähnigen Liganden vorgestellt, die unter aeroben Bedingungen Benzylalkohole und Allylalkohole katalytisch zu den entsprechenden Aldehyden oder Ketonen zu oxidieren vermögen, wobei gleichzeitig Wasserstoffperoxid gebildet wird. Bei einer der besonders wirksamen $Cu^{II}$-Verbindungen, deren Herstellung allerdings recht aufwendig ist, handelt es sich gemäß Chem. & Eng. News vom 26.01.1998, Seite 9 um eine Verbindung folgender Formel:

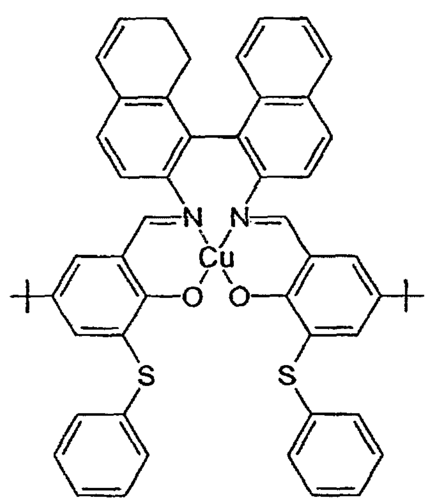

Aufgabe der vorliegenden Erfindung ist es, weitere $Cu^{II}$-Phenoxylkomplexe und daraus erhältliche $Cu^{II}$-Phenoxylradikalkomplexe aufzuzeigen, die Oxidationsreaktionen mit molekularem Sauerstoff katalysieren. Die neuen Cu-Phenoxylradikalkomplexe sollten einfach zugänglich sein. Bevorzugte Cu-Phenoxylradikalkomplexe sollten auch andere Alkohole zu oxidieren gestatten, als dies mit den vorbekannten Komplexen möglich war. Eine weitere Aufgabe richtet sich darauf, für Substrat-Katalysator-Systeme Verwendungen aufzuzeigen, wobei das in situ gebildete Wasserstoffperoxid gezielt genutzt wird.

Gefunden wurden vierzähnige $Cu^{II}$- oder $Zn^{II}$-Phenoxylkomplexe und Phenoxylradikalkomplexe hiervon, worin pro Metallatom ein Ligand L gebunden ist und $H_2L$ für die allgemeine Formel

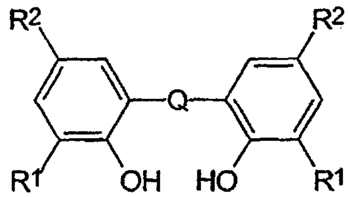

(III)

steht, in welcher Q für ein Brückenglied aus der Reihe -S-, -O-, -N($R^3$)-, -P($R^4$)- oder für ein Brückenglied der Formel ortho-NH-$C_6H_4$-NH-, $R^1$ und $R^2$, die gleich oder verschieden sein können, für radikalstabilisierende Reste, insbesondere Alkyl mit einem am Phenolatring gebundenen tertiären C-Atom, $R^3$ und $R^4$ für H oder eine $C_1$- bis $C_6$-Alkylgruppe stehen.

Ein bevorzugter $Cu^{II}$-Phenoxylradikalkomplex ist zweideutig und weist die allgemeine Formel I auf

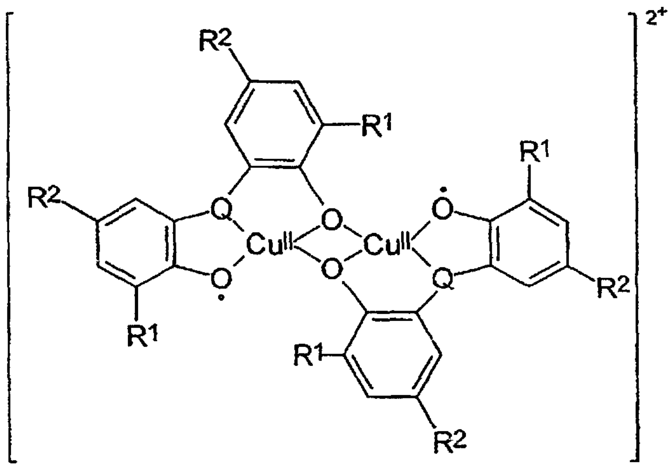

$\frac{2}{n}X^{n-}$ (I),

worin Q für ein Brückenglied aus der Reihe -S-, -O-, -N(R3)- und -P($R^4$)- und X für ein Anion mit der Wertigkeit n stehen und $R^1$, $R^2$, $R^3$ und $R^4$ die vorgenannte Bedeutung haben.

Komplexe der Formel I, worin das Brückenglied Q eine Thioetherbrücke darstellt, werden wegen ihrer höheren Stabilität gegenüber Komplexen mit einer Etherbrücke oder Amin- oder Phosphinbrücke bevorzugt. $R^3$ und $R^4$ im -N($R^3$)-beziehungsweise -P($R^4$)-Brückenglied kann Wasserstoff oder eine lineare, verzweigte oder zyklische Alkylgruppe mit 1 bis 6 C-Atomen sein, beispielsweise Methyl, Ethyl, n-Propyl, iso-Propyl, n-Butyl, sek.-Butyl, Cyclopentyl. Die radikalstabilisierenden Reste $R^1$ und $R^2$ nehmen die ortho- und para-Position zum phenolischen Sauerstoffatom ein. Lineare und bevorzugt verzweigte Alkylgruppen, insbesondere solche mit einem am Phenolatring gebundenen tertiären C-Atom, wie tert.-Butyl, tert.-Amyl und tert.-Hexyl, sind bekannte Radikalstabilisatoren. Zur Radikalstabilisierung und somit als $R^1$ und/oder $R^2$ sind auch Alkylthio- und Arylthiogruppen, wie zum Beispiel i-Propylthio- und Phenylthio, geeignet. Das Anion X mit der Wertigkeit n ist mit der Maßgabe beliebig, daß es nicht selbst zu stark mit dem $Cu^{II}$-Ion koordiniert. X wird vorzugsweise ausgewählt aus der Reihe Chlorid, Bromid, Acetat, Sulfit, Sulfat. Besonders bevorzugte zweikernige $Cu^{II}$-Phenoxylradikalkomplexe der Formel I sind solche, in welchen Q für eine Thioetherbrücke, $R^1$ und $R^2$ für tert.-Alkyl, insbesondere tert.-Butyl, und X für Chlorid steht.

Erfindungsgemäße zweikernige $Cu^{II}$-Phenoxylradikalkomplexe der Formel I sind entweder aus der Umsetzung eines $Cu^{I}$-Salzes der Formel $Cu^{I}_{n}X$, worin X und n die eingangs genannte Bedeutung haben, mit einem dreizähnigen phenolischen Liganden der allgemeinen Formel III, in welchem jedoch Q ungleich ortho-NH-$C_6H_4$-NH- ist, in Gegenwart einer N-haltigen Base B, insbesondere eines tertiären Amins, wie Tri($C_1$- bis $C_4$)alkylamin, mit nachfolgender Behandlung mit trockenem Sauerstoff oder einem trockenen $O_2$-enthaltenden Gas oder durch Behandlung des als Zwischenprodukt entstehenden einkernigen $Cu^{II}$-Phenoxylkomplexes der allgemeinen Formel II mit trockenem Sauerstoff oder einem $O_2$-enthaltenden Gas erhältlich.

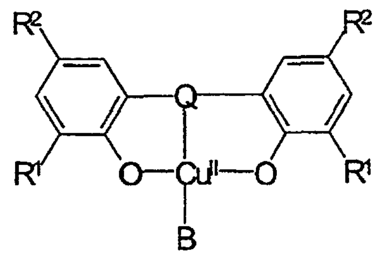

(II)

Die $Cu^{II}$-Phenoxylkomplexe der Formel II sind paramagnetisch, die $Cu^{II}$-Phenoxylradikalkomplexe der Formel I dagegen diamagnetisch. Aufgrund des Elektronenspektrums ist in I die Anwesenheit von Phenoxylradikalen gesichert; das Resonanz-Raman-Spektrum spricht für die zweikernige Struktur von I mit zwei verbrückten Phenolatliganden und zwei Phenoxylradikalen.

Gefunden wurden ferner einkernige Phenoxylkomplexe der allgemeinen Formel $[M(L)]^0$ (Va) und einkernige Phenoxylradikalkomplexe hiervon der allgemeinen Formeln $[M(L)]_n^+X^{n-}$ (Vb) und $[M(LH_2)]_n^+X^{n-}$ (Vc), worin M für $Cu^{II}$ oder $Zn^{II}$ und L für einen vierzähnigen Liganden stehen, wobei $H_4L$ die allgemeine Formel IV (entsprechend Formel III mit Q = ortho-NH-$C_6H_4$-NH-) aufweist:

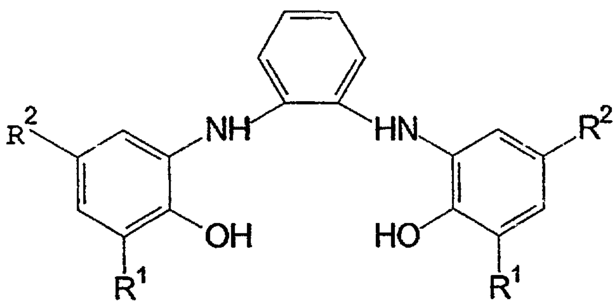

(IV)

$R^1$ und $R^2$ stehen in IV wieder (wie in III) für radikalstabilisierende Gruppen. Besonders bevorzugt ist $R^1 = R^2$ = tert. -Butyl.

Die allgemeine Formel VI zeigt beispielhaft die Struktur eines bevorzugten neutralen $Cu^{II}$-Phenoxylkomplexes des Typs Va mit tert.-Butyl für $R^1$ und $R^2$.

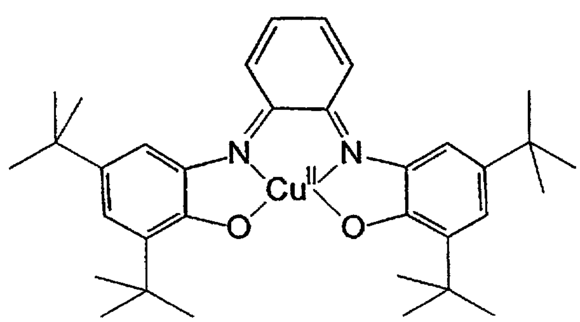

(VI)

Die erfindungsgemäßen neutralen $M^{II}$-Phenoxylkomplexe (Va) sind erhältlich aus der Umsetzung eines $Cu^I$-Salzes oder bevorzugt Komplexes davon beziehungsweise eines $Zn^{II}$-Salzes mit einer phenolischen Verbindung der allgemeinen Formel IV in Gegenwart einer Base mit nachfolgender Behandlung mit trockenem Sauerstoff oder einem trockenen $O_2$-enthaltenden Gas. Geeignete Cu- beziehungsweise Zn-Verbindungen sind beispielsweise $[Cu^I(CH_3CN)_4]ClO_4$, $[Cu^I(NR_3)_4]Cl$ beziehungsweise $Zn(BF_4)_2 \cdot H_2O$, die auch in situ gebildet werden können.

Die zentrale o-Phenylendiamingruppe in $H_4(L)$ (= IV) kann zum Iminosemichinon $H_3(L)$ und weiter zum Diiminochinon $H_2(L)$ oxidiert werden; schließlich können auch die beiden phenolischen Gruppen durch Einelektronenschritte zu H(L) und (L) oxidiert werden. Die fünf Oxidationsstufen der vierzähigen Liganden sind, wie aus dem nachfolgenden Schema, worin auch die Farben der Komplexe angegeben sind, hervorgeht, in den entsprechenden $Cu^{II}$- und $Zn^{II}$-Komplexen zugänglich:

| | $M = Cu^{II}$ | $M = Zn^{II}$ |
|---|---|---|
| $[M^{II}(L)]^{2+}$ | rot | rot |
| -e ⇅ +e | | |
| $[M^{II}(L)]^{+}$ | violett | grün |
| -e ⇅ +e | | |
| $[M^{II}(L)]^{0}$ | grün | blau |
| -e ⇅ +e | | |
| $[M^{II}(LH_2)]^{+}$ | blau | grün |
| -e ⇅ +e | | |
| $[M^{II}(LH_2)]^{0}$ | gelb | farblos |

Die oben genannten Komplexe entstehen beispielsweise aus Komplexen der allgemeinen Formel $[M(L)]^0$ durch elektrochemische Oxidation beziehungsweise Reduktion. Radikalkomplexe mit dem Kation $[M^{II}(L)]^+$ lassen sich auch aus $[M^{II}(L)]^0$ durch Oxidation mit beispielsweise Ferrocenium-hexafluorphosphat gewinnen. Komplexe mit dem Kation $[M^{II}(L)]^+$ oxidieren primäre Alkohole zu Aldehyden, und es entsteht ein Phenoxylradikalkomplex mit dem Kation der Formel $[M(LH_2)]^+$. Komplexe vom Typ $[M^{II}(LH_2)]^+$ werden in saurer Lösung mit Sauerstoff unter Bildung von Wasserstoffperoxid zu $[M^{II}(L)]^+$ oxidiert.

Zur Herstellung der $Cu^{II}$-Phenoxylkomplexe der Formel II, die als gut isolierbare Zwischenprodukte bei der Herstellung der $Cu^{II}$-Phenoxylradikalkomplexe der Formel I selbst Gegenstand der Erfindung sind, werden ein $Cu^{I}$-Salz und ein dreizähniger phenolischer Ligand $LH_2$ der Formel III, worin Q ungleich ortho-NH-$C_6H_4$-NH- ist, in Gegenwart einer stickstoffhaltigen Base B, die als Säureakzeptor für die phenolischen Protonen und zusätzlich als Ligand im Komplex der Formel II wirkt, in einem trockenen Lösungsmittel, zweckmäßigerweise unter Schutzgasatmosphäre, umgesetzt und anschließend mit einem $O_2$ enthaltenden Gas behandelt. Pro Äquilavent $Cu^{I}$ werden mindestens 1 Mol $LH_2$ und mindestens 3 Äquivalente Base eingesetzt. Zur Herstellung von besonders bevorzugten Komplexen der Formel II mit Q gleich Thio und $R^1$ und $R^2$ gleich tert.-Butyl werden CuCl, 2,2'-Thiobis(2,4-di-tert.-butylphenol) und ein Trialkylamin im Molverhältnis von 1 zu gleich oder größer 1 zu gleich oder größer 3, vorzugsweise 1:1:5 eingesetzt. Besonders geeignete Lösungsmittel sind Methanol und offenkettige oder cyclische Ether.

In analoger Weise lassen sich aus dem vierzähnigen Liganden $LH_4$ der Formel IV und Cu- oder Zn-salzen neutrale Komplexe gemäß Va herstellen.

Bei der Lösungsmittelauswahl zur Herstellung der Komplexe ist wesentlich, daß diese nicht mit $Cu^{II}$ oder $Zn^{II}$ koordinieren. Unter dem Begriff "nicht koordinieren" wird verstanden, daß die Koordinationsneigung wesentlich geringer ist als diejenige des Phenolats. Wegen seiner koordinierenden Wirkung ist beispielsweise Acetonitril ungeeignet. Besonders geeignet sind Methanol sowie offenkettige und cyclische Ether.

Unter den Basen B sind Trialkylamine, wie Trimethylamin, Triethylamin, Tri-n-propylamin, Methyl-diethylamin, Dimethyl-ethylamin, Tri-n-butylamin, N-Methylpiperidin, N-methyl-morpholin, und heterocyclische Basen, wie Pyridin, Picoline und N-Methylpyrrol, geeignet. Die Verwendung primärer und sekundärer Amine als Base wird weniger bevorzugt, da diese Basen, wie noch gezeigt wird, im weiteren Verlauf selbst von Radikalen der Formel I oxidiert werden können. Die Umsetzung erfolgt im allgemeinen bei 20 bis 100 °C und insbesondere bei 40 bis 60 °C. Die erhaltene gelbe Lösung wird anschließend, vorzugsweise bei 0 bis 50 °C, insbesondere 10 bis 30 °C, mit einer kontrollierten Menge trockenen Sauerstoffs oder eines $O_2$-haltigen trockenen Gases, wie Luft, behandelt.

Bei der Herstellung von II färbt sich die Lösung tiefblau und nach längerem Stehen fällt der Komplex der Formel II in Form blauer Kristalle aus. In analoger Weise lassen sich die Phenoxyl- und Phenoxylradikalkomplexe von $Cu^{II}$ und $Zn^{II}$ mit dem Liganden aus $H_4(L)$ (= IV) gewinnen. $[Cu^{II}(L)]^0$ (VI) bildet grüne Kristalle und der entsprechende $[Zn^{II}(L)]^0$-Komplex anthrazitfarbene mikrokristalline Kristalle.

Der einkernige $Cu^{II}$-Phenoxylkomplex der Formel II kann in den $Cu^{II}$-Phenoxylradikalkomplex der Formel I überführt werden, und zwar durch Auflösen von II in einem trockenen, nicht mit $Cu^{II}$-koordinierenden Lösungsmittel bei einer Temperatur im Bereich von vorzugsweise 0 bis 50 °C, insbesondere bei Raumtemperatur, und intensives

Kontaktieren mit trockenem Sauerstoff oder einem sauerstoffhaltigen trockenen Gas, wobei sich die blaue Lösung tiefgrün färbt und ein grüner Niederschlag ausfällt.

Gemäß einer weiteren Ausführungsform lassen sich katalytisch wirksame Phenoxylradikalkomplexe auch unmittelbar aus dem $Cu^{I}$- oder $Zn^{II}$-Salz, dem phenolischen Liganden III beziehungsweise IV und einer Base B gewinnen, indem nach der Umsetzung der Reaktionspartner (= Stufe i) die Behandlung mit trockenem $O_2$ oder einem $O_2$haltigen trockenen Gas (= Stufe ii) derart intensiv durchgeführt wird, daß die zunächst sich blaufärbende Lösung tiefgrün wird. Bei dieser Ausführungsform werden pro Äquivalent $Cu^{I}$ mindestens 1 Mol eines phenolischen Liganden und mindestens 2, vorzugsweise mehr als 2 Äquivalente Base eingesetzt. Bevorzugte Lösungsmittel sind lineare und cyclische Ether. Während Aceton als Lösungsmittel gleichfalls geeignet ist, kommt es bei Verwendung von beispielsweise Cyclohexanon, offensichtlich als Folge der Enolisierung, zu Störungen. Zweckmäßigerweise erfolgen auch hier die Umsetzung und die Oxidation unter den zuvor genannten Temperaturbedingungen.

Es wurde gefunden, daß eine $O_2$-freie grüne Lösung eines $Cu^{II}$-Phenoxylradikalkomplexes der Formel I in trockenem THF, zu der bei 20 °C unter Inertgasatmosphäre (Ar) überschüssiges Ethanol gegeben wird, blau färbt, wobei ein Äquivalent Acetaldehyd gebildet wird.

$$[(Cu^{II}L)_2]^{2+} + C_2H_5OH \rightarrow [Cu^{II}L)_2] + CH_3\text{-}CHO + 2\,H^+ \qquad (1)$$

Die Begasung der blauen Lösung mit molekularem Sauerstoff führt innerhalb weniger Minuten wieder zu einer tiefgrünen Lösung, wobei unter Bildung von einem Äquivalent Wasserstoffperoxid der Radikalkomplex zurückgebildet wird.

$$[(Cu^{II}L)_2] + O_2 + 2\,H^+ \rightarrow [Cu^{II}L)_2]^{2+} + H_2O_2 \qquad (2)$$

Die Kombination der Gleichungen (1) und (2) ergibt im Prinzip die homogen katalysierte Oxidation von Ethanol zu Acetaldehyd mit $O_2$ unter Bildung von $H_2O_2$. In analoger Weise lassen sich in Gegenwart von I auch andere primäre Alkohole, ausgenommen Methanol, in die entsprechenden Aldehyde überführen. Als Nebenprodukte entstehen als Folge einer nach einem anderen Reaktionsmechanismus ablaufenden C-C-Verknüpfung symmetrische vicinale Diole sowie Folgeprodukte derselben, nämlich $\alpha$-Hydroxyketone und $\alpha$-Diketone. Aus sekundären Alkoholen entstehen Ketone und/oder symmetrische vicinale Diole und gegebenenfalls deren zuvor genannten Folgeprodukte. Bisher ist noch nicht bekannt, welche Parameter des zu oxidierenden primären beziehungsweise sekundären Alkohols für die Überführung in den entsprechenden Aldehyd oder Keton und welche für die Überführung in ein symmetrisches vicinales Diol verantwortlich sind. Während das Hauptprodukt der Oxidation von Isopropanol Pinakol ist, wird 2-Butanol nahezu quantitativ zu 2-Butanon oxidiert. Unter Verwendung eines eingesetzten oder in situ im Reaktionsmedium gebildeten erfindungsgemäßen $Cu^{II}$-Phenoxylradikalkomplexes der Formel I lassen sich primäre oder sekundäre aliphatische, cycloaliphatische, aromatisch-aliphatische sowie olefinisch ungesättigte oder andere Substituenten enthaltende Alkohole oxidieren.

In analoger Weise lassen sich auch primäre und sekundäre Amine unter Verwendung des erfindungsgemäßen Katalysators der Formel I oder seiner Vorstufe II oxidieren: Aus primären Aminen entstehen Imine beziehungsweise deren Hydrolyseprodukte, also Aldehyde oder Ketone. Aus sekundären Aminen entstehen Schiff'sche Basen. Auch bei primären und sekundären Aminen können als Folge einer Oxidation mit C-C-Verknüpfung symmetrische vicinale Diamine oder Folgeprodukte derselben, wie $\alpha$-Diketone, gebildet werden. Der Fachmann wird im Einzelfall durch einen Vorversuch ermitteln, welches Produkt Hauptprodukt ist und wie das gesamte Produktspektrum aussieht.

Auch die erfindungsgemäßen einkernigen Phenoxylradikalkomplexe der Formel $[M^{II}(L)]^+X^-$ (Vb), die ihrerseits durch Oxidation der neutralen Phenoxylkomplexe $[M^{II}(L)]$ (Va) oder in situ aus einer Metallverbindung und einer Verbindung $LH_4$ gemäß Formel IV zugänglich sind, sind besonders geeignet als Oxidationskatalysatoren zur Oxidation von primären Alkoholen zu Aldehyden mit Luft unter gleichzeitiger Bildung von Wasserstoffperoxid. Die Figur zeigt ein Schema dieses Kreisprozesses. Sekundäre Alkohole werden mit diesem Katalysatorsystem praktisch nicht oxidiert.

Gemäß einer bevorzugten Ausführungsform der Oxidation alkoholischer oder aminischer Substrate werden diese zu einer den $Cu^{II}$-Phenoxylradikalkomplex der Formel I oder einer Vorstufe desselben in katalytischer Menge enthaltenden Lösung, beispielsweise in einem Ether, gegeben und unter Luft- oder $O_2$-Zufuhr gerührt. Zweckmäßigerweise liegt das molare Verhältnis Substrat zu Katalysator im Bereich von 10 bis 10000, insbesondere 100 bis 1000. Oxidationsprodukt des Substrats und $H_2O_2$ lassen sich mittels extraktiver Methode voneinander trennen und dann aufarbeiten. Analog lassen sich primäre Alkohole unter Einsatz oder in situ-Bildung eines erfindungsgemäßen einkernigen $Cu^{II}$- oder $Zn^{II}$-Phenoxylradikalkomplexes $[M(L)]^+X^-$ (Vb) oxidieren.

Erfindungsgemäße Phenoxylradikal-Katalysatoren lassen sich in Verbindung mit einem alkoholischen Substrat auch derart verwenden, daß das im System Substrat-Katalysator durch Oxidation mit Luft gebildete Wasserstoffperoxid zu Bleich-, Reinigungs- und Desinfektionszwecken genutzt wird.. Substrate sind hier beispielsweise Zucker, natürlich vorkommende Zuckeralkohole und natürlich vorkommende Hydroxycarbonsäuren, wie Milchsäure und Weinsäure. Das gebildete Wasserstoffperoxid kann Farbstoffe oxidieren und diese dabei entfärben.

Die erfindungsgemäß katalysierte Oxidationsreaktion von primären und sekundären Alkoholen oder Aminen führt im allgemeinen nicht über die Stufe der genannten Carbonyloder Iminverbindungen heraus. Primäre Alkohole werden also nicht in die Carbonsäuren überführt. Dies ist ein besonderer Vorteil gegenüber anderen Oxidationsreaktionen. Ein weiterer Vorteil ist, daß die Oxidation im wesentlichen unter neutralen und sehr milden Bedingungen abläuft, so daß auch Alkohole mit säure- oder baseempfindlichen Substituenten oder thermisch empfindlichen Strukturen oxidiert werden können. Die Oxidation eignet sich damit auch zur Oxidation von Naturstoffen sowie säure- oder baseempfindliche Schutzgruppen aufweisenden Verbindungen.

Beispielsweise läßt sich Retinol in hoher Ausbeute in Retinal überführen und Glycerin in Glycerinaldehyd, wobei das Glycerin in ketalisierter Form oxidiert wird. Das bei erfindungsgemäßer Verwendung gebildete Wasserstoffperoxid läßt sich unter Einsatz billiger Substrate leicht erhalten und zu Bleich-, Reinigungs- und Desinfektionszwecken verwenden.

**Beispiel 1:** Herstellung des Komplexes der Formel II mit Q = -S-, $R^1$=$R^2$=tert.-Butyl, B = Triethylamin

Der Ligand $LH_2$ der Formel III wurde nach S. Pastor et al., J. Heterocycl. Chem. (1984), 21, Seite 1285, hergestellt. Eine Suspension von CuCl (0,10 g; 1,0 mmol), $LH_2$ (0,44 g; 1,0 mmol) und $NEt_3$ (0,5 mL) in trockenem, $O_2$-freiem $CH_3OH$ (50 mL) wurde 1 h unter Rückfluß in einer Ar-Schutzgasatmosphäre erhitzt. Die auf 20 °C abgekühlte klare gelbe Lösung wurde unter Rühren der Luft ausgesetzt, woraufhin die Lösung sich tiefblau färbte. Nach 2 Tagen fielen blaue Mikrokristalle des genannten Komplexes der Formel II aus. Umkristallisation aus $CH_3OH$/n-Pentan (2:1 Vol) ergibt blaue rhombische Kristalle. Ausbeute: 0,48 g; 80 %.

**Beispiel 2:** Herstellung des Komplexes der Formel I mit Q = -S-, $R^1$=$R^2$=tert.-Butyl, X = Cl

Eine Suspension von CuCl, $LH_2$, $NEt_3$ (Mengen wie in Beispiel 1) in trockenem THF wurde 30 min unter Rückfluß in einer Ar-Atmosphäre erhitzt. Durch die auf 20 °C abgekühlte, klare gelbe Lösung wurde unter Rühren 1 h trockener, reiner Sauerstoff geleitet ($H_2O$-Ausschluß ist wichtig). Die Lösung färbt sich erst blau, dann tiefgrün und es fällt ein mikrokristalliner grüner Niederschlag des genannten Radikalkomplexes der Formel I aus. Umkristallisation aus trockenem THF. Ausbeute: 0,12 g; 11 %.

**Beispiele 3 bis 9:** Oxidation primärer und sekundärer Alkohole und primärer Amine in Gegenwart eines zweikernigen Phenoxylradikalkomplex (I)

Je $1{,}25 \cdot 10^{-5}$ Mol CuCl und Ligand $LH_2$ (mit Q = S und $R^1$=$R^2$=tert.-Butyl) und $2{,}5 \cdot 10^{-5}$ Mol Triethylamin wurden in trockenem THF (50 mL) unter Ar-Schutzgas bei 50 °C gelöst. Anschließend wurden $6{,}25 \cdot 10^{-3}$ Mol des jeweiligen Alkohols zugegeben und unter Luftzutritt 12 h bei 20 °C gerührt. Das Reaktionsgemisch wurde gaschromatographisch analysiert; das $H_2O_2$ wurde spektralphotometrisch nach Komplexierung mit Titanylsulfat in wäßriger $H_2SO_4$ bestimmt. Die Substrate und erhaltenen Produkte folgen aus der Tabelle.

Benzylamin und Aminoethanol wurden analog der Oxidation unterworfen. Die Ergebnisse folgen aus der Tabelle.

Tabelle

| Beispiel Nr. | Substrat | Produkte (% Ausbeute, bez. auf den Alkohol) | |
|---|---|---|---|
| 3 | Ethanol | Acetaldehyd | (63) |
| | | Butan-2,3-diol | (1,5) |
| | | 3-Hydroxy-butanon-2 | (1,5) |
| | | Butan-2,3-dion | (4) |
| | | $H_2O_2$ | (70) |

Tabelle (fortgesetzt)

| Beispiel Nr. | Substrat | Produkte (% Ausbeute, bez. auf den Alkohol) | |
|---|---|---|---|
| 4 | Benzylalkohol | Benzaldehyd | (60) |
| | | 1,2-Diphenylethandiol | (1) |
| | | Benzoin | (1) |
| | | Benzil | (3) |
| | | $H_2O_2$ | (65) |
| 5 | Isopropanol | Aceton | (2) |
| | | Pinakol | (61) |
| | | $H_2O_2$ | (61) |
| 6 | Diphenylcarbinol | Tetraphenylethandiol | (68) |
| | | $H_2O_2$ | (66) |
| 7 | Isobutanol | Butan-2-on | (70) |
| | | $H_2O_2$ | (70) |
| | | Benzaldehyd | (~ 20 %) |
| | | $H_2O_2$ | (~ 20 %) (nicht optimiert) |
| 9 | Aminoethanol | Glyoxal | (quantitativ) |
| | | $H_2O_2$ | (quantitativ) |

**Beispiele 10 bis 15**

In situ-Bildung des Katalysators, worin Q für -NH und $R^1$ und $R^2$ für tert.-Butyl stehen, und Oxidation von Alkoholen.

Zu jeweils 50 ml einer THF-Lösung enthaltende je $2,5 \cdot 10^{-4}$ Mol/l CuCl und NH-Ligand (2,2'-Aminobis(2,4-di-tert.butylphenol) und $(2,5 \text{ bis } 25)10^{-4}$ Mol/l Triethylamin wurden 6,25 mMol Substrat zugegeben und das Gemisch unter Luftzutritt gerührt. Die Reaktionsgemische werden analysiert. Die Substrate und Ergebnisse folgen aus der Tabelle.

Tabelle

| Beispiel Nr. | Substrat | Ausbeute | |
|---|---|---|---|
| | | Aldehyd (%) | $H_2O_2$ (%) |
| 10 | Ethanol | Acetaldehyd (66) | (68) |
| 11 | Benzylalkohol | Benzaldehyd (72) | (74) |
| 12 | 1-Hexanol | 1-Hexanol (52) | (52) |
| 13 | Isopropanol | keine Oxidation | |
| 14 | 2-Butanol | keine Oxidation | |
| 15 | Cyclohexanol | keine Oxidation | |

Mit dem eine Aminbrücke aufweisenden Katalysator lassen sich primäre Alkohole gut oxidieren, nicht jedoch sekundäre Alkohole.

Unter Bedingungen analog Beispiel 1, jedoch unter Einsatz des oben genannten aminischen Liganden wurde der entsprechende Komplex der Formel II mit Q = -NH- hergestellt. Analyse und Kristallstrukturanalyse sprechen für den mononuklearen Neutralkomplex der Formel II.

**Beispiel 16:** Herstellung von NN'-Bis(3,5-di-tert.butyl-2- hydroxyphenyl)-1,2-phenylendiamin (= Ligand $H_4L$ der Formel IV)

Eine Suspension von 3,5-Di-tert.-butylcatechol (8,88 g; 40 mmol), o-Phenylendiamin (2,1 g; 20 mmol) und Triethylamin (0,4 ml) in n-Heptan (120 ml) wurde 4 Tage an der Luft gerührt. Der beige Niederschlag wurde isoliert und mit Pentan gewaschen. Ausbeute: 4,6 g; 45 %.

**Beispiel 17:** Herstellung des Cu-Komplexes $[Cu^{II}(L)]^0$ gemäß Formel VI

Eine Lösung von $[Cu(CH_3CN)_4]$ $(ClO_4)$ (1 mmol; 0,327 g), $H_4L$ (= IV) gemäß Beispiel 14 (1 mmol) und Triethylamin (0,5 ml) in trockenem, $O_2$-freiem Methanol (50 ml) wurde 0,5 h unter Rückfluß einer Ar-Schutzgasatmosphäre erhitzt. Die auf 20 °C abgekühlte gelbe Lösung wurde der Luft ausgesetzt, woraufhin die Lösung sich tiefgrün färbte. Nach 2 h fielen grüne Mikrokristalle des Komplexes der Formel (V) aus. Umkristallisation aus Acetonnitril ergibt grüne Kristalle. Ausbeute: 0,28 g; 49 %.

**Beispiel 18:** Herstellung des Zn-Komplexes $[Zn^{II}(L)]^0$ (analog VI)

Eine Lösung von $Zn(BF_4)_2 \cdot 2H_2O$ (1 mmol; 0,24 g), $H_4L$ (= IV) gemäß Beispiel 14 (1 mmol; 0,516 g) und $NEt_3$ (0,5 ml) in trockenem Methanol (50 ml) wurde unter Argon 0,5 h erhitzt. Die auf Raumtemperatur abgekühlte gelbe Lösung wurde der Luft ausgesetzt. Die Lösung färbte sich tiefgrün und es fiel innerhalb eines Tages ein anthrazitfarbener mikrokristalliner Komplex aus. Ausbeute: 0,32 g; 62 %.

**Beispiel 19:** Oxidation von Methanol

Je 0,25 mmol CuCl und Ligand $H_4L$ (= IV) und Triethylamin (10 µl) wurden in trockenem THF (50 ml) unter Ar-Schutzgas für 10 Min. gerührt. Anschließend wurden 253 µl abs. Methanol zugegeben und unter Luftzutritt 24 h bei 20 °C gerührt. Das Reaktionsgemisch wurde gaschromatographisch analysiert; das $H_2O_2$ wurde spektralphotometrisch nach Komplexierung mit Titanylsulfat bestimmt. Produkte: Formaldehyd 60 % / 23 h; $H_2O_2$ 65 % / 23 h.

**Beispiel 20:** Herstellung des Komplexes $[M^{II}(LH_2)]^0$ mit M = $Cu^{II}$ oder $Zn^{II}$ (entsprechend Vc)

$Zn(ClO_4)_2 \cdot 6H^2O$ oder $Cu(ClO_4)_2 \cdot 6H_2O$ wird in $O_2$-freier methanolischer Lösung in Gegenwart eines tertiären Amins unter Ar-Schutzgas umgesetzt. Der Komplex fällt als gelbbrauner beziehungsweise brauner Niederschlag aus.

**Beispiel 21:** Herstellung des Komplexes $[M^{II}(L)]PF_6$ mit M = $Cu^{II}$ oder $Zn^{II}$ (entsprechend Vb)

Eine Lösung des Komplexes aus Beispiel 15 oder 16 in $CH_2Cl_2$ wurde mit Ferrocenium-hexafluorphosphat bei -10 °C umgesetzt und bei -20 °C (Cu-Komplex) beziehungsweise -80 °C (Zn-Komplex) auskristallisiert. Der Cu-Komplex fällt als violetter Niederschlag, der Zn-Komplex als grüner Niederschlag aus.

## Patentansprüche

1. Vierzähnige $Cu^{II}$- oder $Zn^{II}$- Phenoxylkomplexe und Phenoxylradikalkomplexe hiervon, worin pro Metallatom ein Ligand L gebunden ist und H2L für die allgemeine Formel

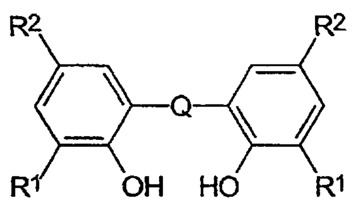

(III)

steht, in welcher Q für ein Brückenglied aus der Reihe -S-, -O-, $-N(R^3)-$, $-P(R^4)-$ oder für ein Brückenglied der Formel ortho-$NH-C_6H_4-NH$-, $R^1$ und $R^2$, die gleich oder verschieden sein können, für radikalstabilisierende Reste, insbesondere Alkyl mit einem am Phenolatring gebundenen tertiären C-Atom, $R^3$ und $R^4$ für H oder eine $C_1$- bis

$C_6$-Alkylgruppe stehen.

**2.** $Cu^{II}$-Phenoxylradikalkomplex nach Anspruch 1, **gekennzeichnet durch** die allgemeine Formel I

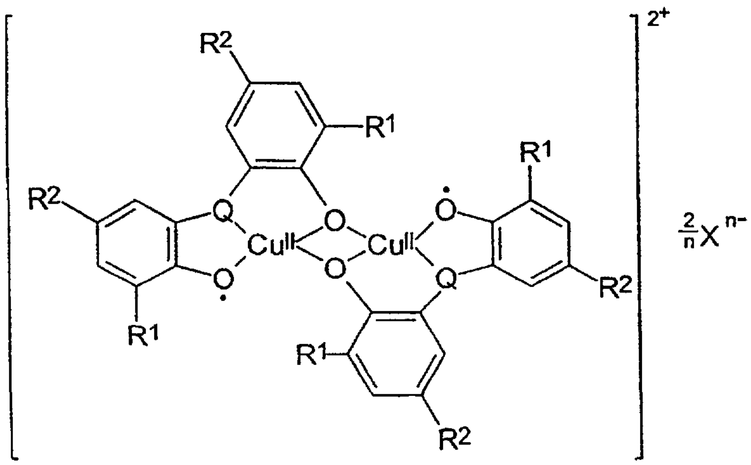

(I),

worin Q für ein Brückenglied aus der Reihe -S-, -O-, $-N(R^3)-$ oder $-P(R^4)-$ und X für ein Anion mit der Wertigkeit n stehen und $R^1$, $R^2$, $R^3$ und $R^4$ die vorgenannte Bedeutung haben.

**3.** $Cu^{II}$-Phenoxylkomplex der allgemeinen Formel II

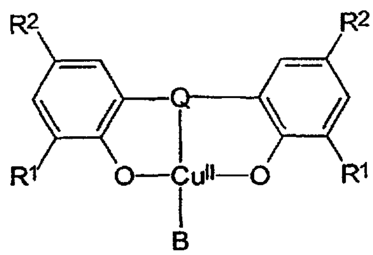

(II),

worin Q für ein Brückenglied aus der Reihe -S-, -O-, $-N(R^3)-$ und $-P(R^4)-$ steht und $R^1$, $R^2$, $R^3$ und $R^4$ die gleiche Bedeutung haben wie in Anspruch 1 und B für eine stickstoffhaltige Base, insbesondere ein tertiäres Amin, steht.

**4.** Phenoxylkomplex und Phenoxylradikalkomplex gemäß Anspruch 1, **gekennzeichnet durch** die allgemeinen Formeln $[M(L)]^0$ (Va), $[M(L)]_n{}^+X^{n-}$ (Vb) und $[M(LH_2)]_n{}^+X^{n-}$ (Vc), worin M für $Cu^{II}$ oder $Zn^{II}$ und L für einen vierzähnigen Liganden stehen, wobei $H_4(L)$ die allgemeine Formel IV

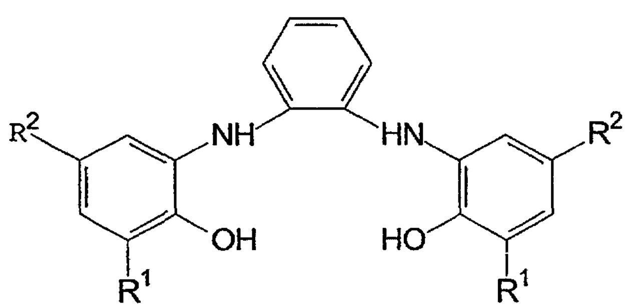

(IV)

aufweist (entsprechend III mit Q = ortho-$NH-C_6H_4-NH$-) und $R^1$ und $R^2$ sowie X und n die vorgenannte Bedeutung

haben.

**5.** Phenoxyl- oder Phenoxylradikalkomplex gemäß einem der Ansprüche 1 bis 4, **dadurch gekennzeichnet, daß** $R^1$ und $R^2$ tert.-Butyl bedeuten.

**6.** Phenoxyl- oder Phenoxylradikalkomplex gemäß einem der Ansprüche 1 bis 3, **dadurch gekennzeichnet, daß** das Brückenglied Q für eine Thioethergruppe und X für Chlorid stehen.

**7.** Verfahren zur Herstellung vierzähniger $Cu^{II}$-oder $Zn^{II}$-Phenoxylkomplexe und Phenoxylradikalkomplexen hiervon gemäß Anspruch 1,
**dadurch gekennzeichnet,**
**daß** es umfaßt (i) Umsetzen eines Metallsalzes oder Metallkomplexes von Kupfer oder Zink mit einer Verbindung der allgemeinen Formel III

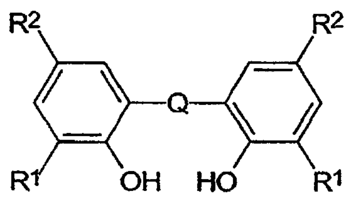

(III)

wobei pro g-Atom Metall mindestens ein Mol Ligand eingesetzt wird, in einem trockenen $O_2$-freien Lösungsmittel, insbesondere einem Ether oder Methanol, in Gegenwart von mindestens zwei g-Äquivalenten einer stickstoffhaltigen Base B, insbesondere eines tertiären Amins, pro g-Atom Metall bei 20 bis 100 °C unter Inertgasatmosphäre, (ii) Kontaktieren der in Stufe (i) erhaltenen Lösung mit trockenem Sauerstoff oder einem $O_2$ enthaltenden trockenen Gas und (iii) Abtrennen des ausgefallenen Niederschlags.

**8.** Verwendung eines $Cu^{II}$- oder $Zn^{II}$-Phenoxyl- oder Phenoxylradikalkomplexes gemäß einem der Ansprüche 1 bis 6 als Katalysator oder Katalysatorvorstufe für Oxidationsreaktionen mittels Sauerstoff unter Bildung von Wasserstoffperoxid.

**9.** Verwendung nach Anspruch 8, wobei man einem Phenoxylradikalkomplex gemäß einem der Ansprüche 1 bis 7 nach dem Verfahren gemäß Stufen (i) und (ii) des Anspruchs 8 in situ bildet und das Gemisch nach Zugabe eines zu oxidierenden Substrats unter Luftzutritt behandelt.

**10.** Verfahren zur Oxidation eines organischen Substrats aus der Reihe primärer oder sekundärer Alkohole und Amine mit Sauerstoff,
**dadurch gekennzeichnet,**
**daß** man einen $Cu^{II}$- oder $Zn^{II}$-Phenoxylradikalkomplex gemäß einem der Ansprüche 1 bis 7 als Oxidationskatalysator einsetzt oder diesen gemäß Stufe (i) und (ii) des Anspruchs 8 in situ bildet und das Substrat in Gegenwart des Katalysators in einem trockenen Lösungsmittel mit $O_2$ oder einem $O_2$ enthaltenden Gas kontaktiert.

## Claims

**1.** Quadra-dentate $Cu^{II}$- or $Zn^{II}$- phenoxyl complexes and phenoxyl radical complexes thereof, in which one ligand L is bonded per metal atom and $H_2L$ represents the general formula

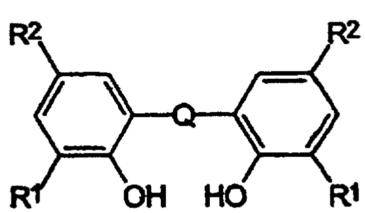

**(III)**

in which Q represents a binding link from the series -S-, -O-, $-N(R^3)-$, $-P(R^4)-$ or a binding link of the formula ortho-$NH-C_6H_4-NH-$, $R^1$ and $R^2$, which may be the same or different, represent radical stabilising groups, in particular alkyl with a tertiary C atom bonded to the phenolate ring, $R^3$ and $R^4$ represent H or a $C_1$- to $C_6$-alkyl group.

**2.** $Cu^{II}$-phenoxyl radical complex according to claim 1, **characterised by** the general formula I

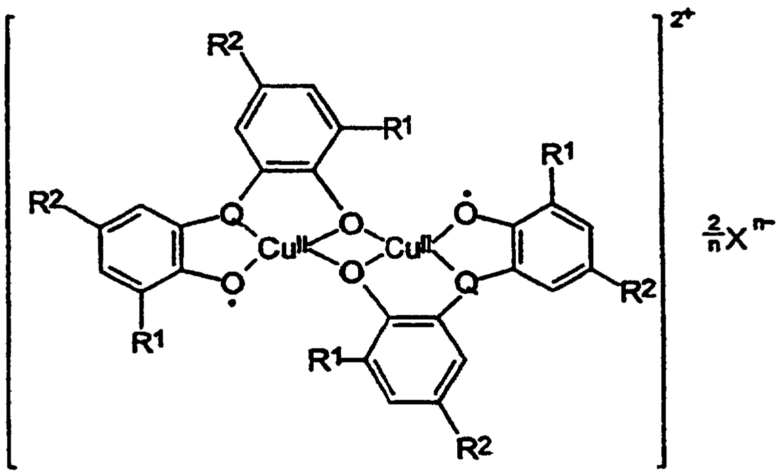

(I),

in which Q represents a binding link from the series -S-, -O-, $-N(R^3)-$ or $-P(R^4)-$, X represents an anion with the valence n and $R^1$, $R^2$, $R^3$ and $R^4$ have the meaning given above.

**3.** $Cu^{II}$-phenoxyl complex of the general formula II

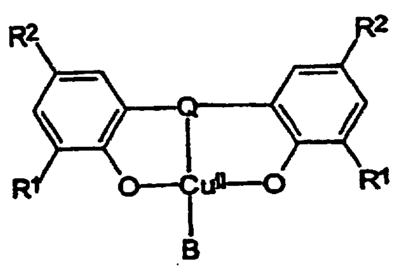

(II),

in which Q represents a binding link from the series -S-, -O-, $-N(R^3)-$ and $-P(R^4)-$ and $R^1$, $R^2$, $R^3$ and $R^4$ have the same meaning as in claim 1, and B represents a nitrogen-containing base, in particular a tertiary amine.

**4.** Phenoxyl complex and phenoxyl radical complex according to claim 1, **characterised by** the general formulae $[M(L)]^0$ (Va), $[M(L)]_n^+X^{n-}$ (Vb) and $[M(LH_2)]_n^+X^{n-}$ (Vc) in which M stands for $Cu^{II}$ or $Zn^{II}$ and L for a quadra-dentate ligand, $H_4(L)$ having the general formula IV

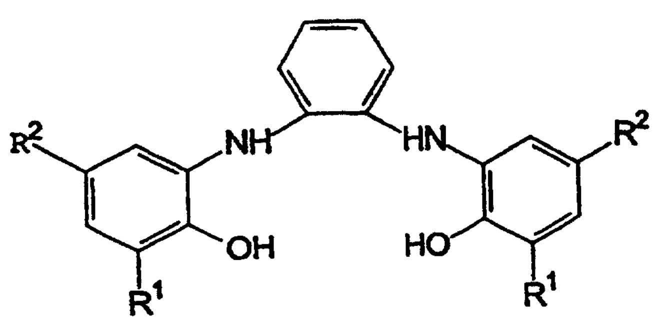

(IV)

(according to III with Q = ortho-NH-$C_6H_4$-NH-) and $R^1$ and $R^2$ and X and n having the meaning given above.

**5.** Phenoxyl- or phenoxyl radical complex according to one of claims 1 to 4, **characterised in that** $R^1$ and $R^2$ mean tert.-butyl.

**6.** Phenoxyl- or phenoxylradical complex according to one of claims 1 to 3, **characterised in that** the binding link Q represents a thioether group and X represents a chloride.

**7.** Process for the production of quadra-dentate $Cu^{II}$- or $Zn^{II}$-phenoxyl complexes and phenoxyl radical complexes thereof according to claim 1, **characterised in that** it comprises (i) reaction of a metal salt or metal complex of copper or zinc with a compound of the general formula III

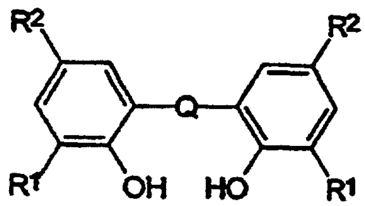

(III)

at least 1 mol of ligand being used per g-atom of metal, in a dry $O_2$-free solvent, in particular an ether or methanol, in the presence of at least two g-equivalents of a nitrogen-containing base B, in particular a tertiary amine, per g-atom of metal at 20 to 100°C under an inert gas atmosphere, (ii) bringing the solution obtained in stage (i) into contact with dry oxygen or an $O_2$-containing dry gas and (iii) separating the precipitated deposit.

**8.** Use of a $Cu^{II}$- or $Zn^{II}$-phenoxyl- or phenoxylradical complex according to one of claims 1 to 6 as a catalyst or catalyst precursor for oxidation reactions by means of oxygen with the formation of hydrogen peroxide.

**9.** Use according to claim 8, wherein a phenoxyl radical complex according to one of claims 1 to 7 is formed in situ by the process according to stages (i) and (ii) of claim 8 and the mixture is treated with the admission of air after adding a substrate to be oxidised.

**10.** Process for the oxidation of an organic substrate from the series of primary or secondary alcohols and amines with oxygen, **characterised in that** a $Cu^{II}$- or $Zn^{II}$-phenoxyl radical complex according to one of claims 1 to 7 is used as an oxidation catalyst or is formed in situ according to stage (i) and (ii) of claim 8 and the substrate is brought into contact with $O_2$ or an $O_2$-containing gas in a dry solvent in the presence of the catalyst.

## Revendications

**1.** Complexes $Cu^{II}$- ou $Zn^{II}$-phénoxyle tétradentés et leurs complexes de radicaux phénoxyle, où par atome de métal un ligand L est lié, et $H_2L$ représente la formule générale

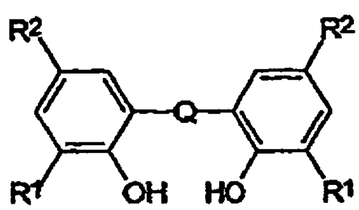

(III)

dans laquelle Q représente un élément de pont de la série -S-, -O-, -N($R^3$)-, -P($R^4$)- ou un élément de pont de formule ortho-NH-$C_6H_4$-NH-, $R^1$ et $R^2$, qui peuvent être identiques ou différents, représentent des radicaux de stabilisation radicalaire, comportant un atome de carbone tertiaire lié sur le noyau phénolate, $R^3$ et $R^4$ représentent H ou un groupe alkyle en $C_1$ à $C_6$.

**2.** Complexe de radical $Cu^{II}$-phénoxy selon la revendication 1,
**caractérisé par**
la formule générale I

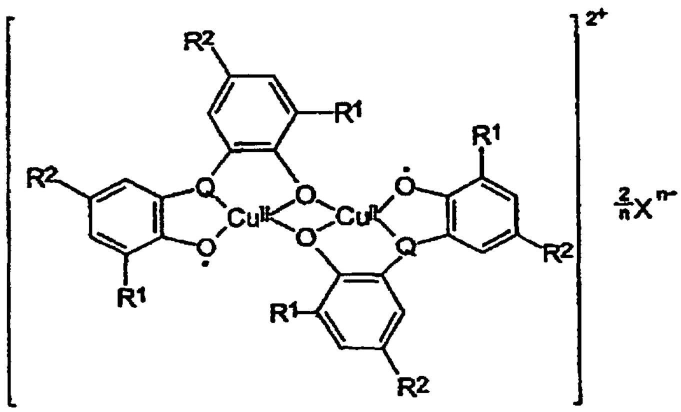

(I),

dans laquelle
Q représente un élément de pont de la série -S-, -O-, -N($R^3$)-, -P($R^4$)- et X représente un anion de valence n, et $R^1$, $R^2$, $R^3$ et $R^4$ ont les significations indiquées ci-dessus.

**3.** Complexe $Cu^{II}$-phénoxyle de formule générale II

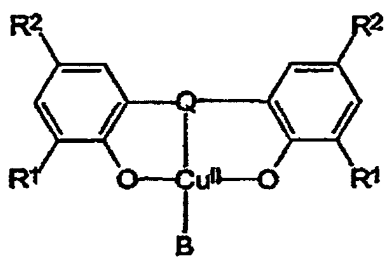

(II),

dans laquelle
Q représente un élément de pont de la série -S-, -O-, -N($R^3$)-, -P($R^4$)- et X représente un anion de valence n, et $R^1$, $R^2$, $R^3$ et $R^4$ ont les mêmes significations que dans la revendication 1 et B représente une base azotée, en particulier une amine tertiaire.

**4.** Complexe de phénoxyle et complexe de radicaux phénoxyles selon la revendication 1,
**caractérisés par**
les formules générales $[M(L)]^0$ (Va), $[M(L)]_n^+X^{n-}$ (Vb) et $[M(LH_2)]_n^+X^{n-}$ (Vc), où M représente $Cu^{II}$ ou $Zn^{II}$ et L représente un ligand, où $H_4(L)$ présente la formule générale IV

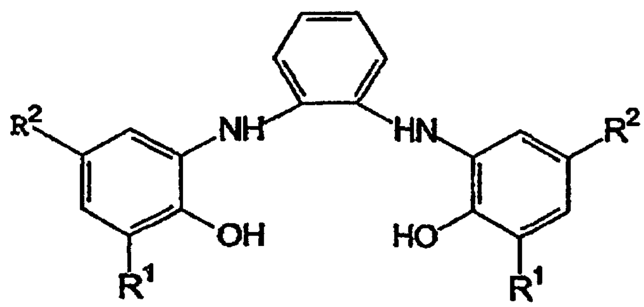

(IV)

(correspondant à III avec Q = ortho-NH-$C_6H_4$-NH-) et $R^1$ et $R^2$ ainsi que X et n ont la signification indiquée ci-dessus.

**5.** Complexe de phénoxyle ou de radical phénoxyle selon l'une des revendications 1 à 4,
**caractérisé en ce que**
$R^1$ et $R^2$ représentent un tert-butyle.

**6.** Complexe de phénoxyle ou de radical phénoxyle selon l'une des revendications 1 à 3,
**caractérisé en ce que**
l'élément de pont Q représente un groupe thioéther et X représente un chlorure.

**7.** Procédé de préparation de complexe de $Cu^{II}$- ou $Zn^{II}$-phénoxyle et de complexe de radical phénoxyle qui en découle selon la revendication 1,
**caractérisé en ce qu'**
il comprend (i) la réaction d'un sel métallique ou d'un complexe métallique de cuivre ou de zinc avec un composé de formule générale III

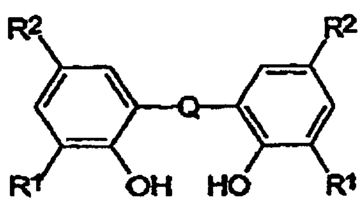

(III)

dans laquelle par atome-gramme de métal on utilise au moins un mole de ligand, dans un solvant sec sans oxygène, en particulier un éther ou le méthanol, en présence d'au moins deux équivalents-grammes d'une base azotée B, en particulier d'une amine tertiaire, par atome-gramme de métal entre 20 et 100°C sous une atmosphère de gaz inerte, (ii) la mise en contact de la solution obtenue dans l'étape (i) avec de l'oxygène sec ou un gaz sec contenant de l'oxygène, et (iii) la séparation du précipité obtenu.

**8.** Utilisation d'un complexe $Cu^{II}$ ou $Zn^{II}$-phénoxyle ou radical phénoxyle selon l'une des revendications 1 à 6 comme catalyseur ou précurseur de catalyseur pour des réactions d'oxydation au moyen d'oxygène avec formation de peroxyde d'hydrogène.

**9.** Utilisation selon la revendication 8, dans laquelle on forme un complexe de radical phénoxyle selon l'une des revendications 1 à 7 selon le procédé des étapes (i) et (ii) de la revendication 8 in situ et l'on traite le mélange avec apport d'air après addition d'un substrat à oxyder.

**10.** Procédé d'oxydation d'un substrat organique de la série des alcools primaires ou secondaires avec des aminés,
**caractérisé en ce qu'**
on utilise un complexe radicalaire $Cu^{II}$ ou $Zn^{II}$-phénoxyle selon l'une des revendications 1 à 7 comme catalyseur d'oxydation, ou bien où l'on forme celui-ci selon les étapes (i) et (ii) des revendications 1 à 8 et on met en contact le substrat en présence de catalyseur dans un solvant sec avec $O_2$ ou un gaz contenant $O_2$.

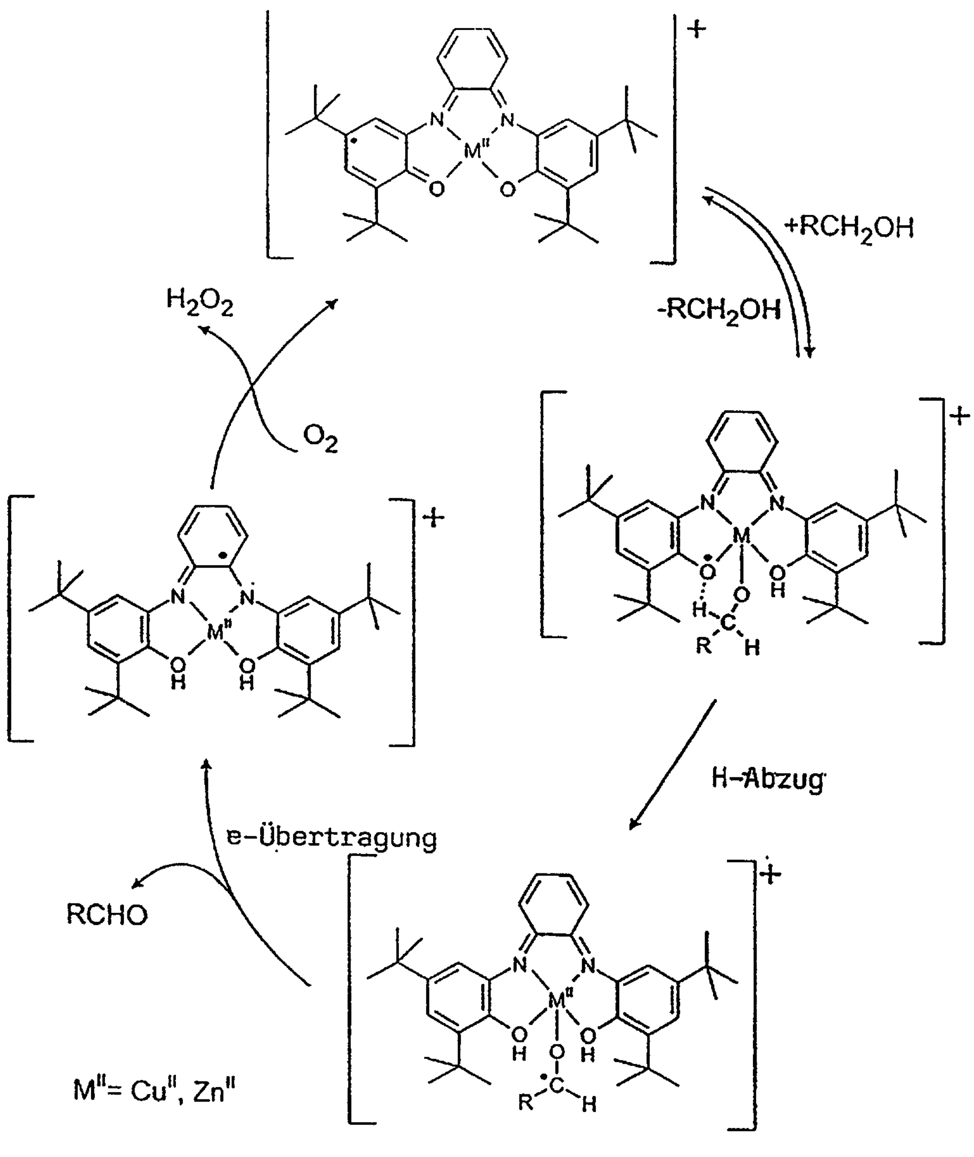


Figur 1/1